# FASCICULE DE BREVET EUROPEEN

(11) **EP 2 813 260 B1**
(45) Date de publication et mention de la délivrance du brevet: **20.07.2016**
(21) Numéro de dépôt: 14163235.6
(22) Date de dépôt: 02.04.2014
(51) Int. Cl.: A61N 1/36

(54) **Dispositif médical implantable actif pour le traitement de l'insuffisance cardiaque avec stimulation stochastique du nerf vague**
Aktives implantierbares medizinisches Gerät zur Behandlung einer Herzinsuffizienz mit stochastischer Stimulation des Vagusnervs
Active implantable medical device for treating heart failure with stochastic stimulation of the vagus nerve

(30) Priorité: 11.06.2013 FR 1355383
(43) Date de publication de la demande: 17.12.2014
(73) Titulaire: Sorin CRM SAS, 92140 Clamart Cedex (FR)
(72) Inventeur: Legay, Thierry, 91640 Fontenay les Briis (FR); Blumstein, Hervé, 28130 Mévoisins (FR)
(74) Mandataire: Dupuis-Latour, Dominique

(56) Documents cités:
- US-A1- 2007 203 527
- US-A1- 2007 233 194
- US-A1- 2009 088 817
- US-A1- 2011 093 026
- US-A1- 2011 118 802
- US-A1- 2013 131 746

## Description

L'invention concerne les "dispositifs médicaux implantables actifs" tels que définis par la directive 90/385/CEE du 20 juin 1990 du Conseil des communautés européennes, plus précisément les implants permettant de délivrer des thérapies de stimulation du nerf vague, dites thérapies VNS (*Vagus Nerve Stimulation*).

Elle concerne plus particulièrement l'utilisation de telles thérapies chez des patients en risque d'insuffisance cardiaque.

En effet, la stimulation du nerf vague agit sur les fonctions cardiovasculaires par réduction du rythme cardiaque et de la contractilité du myocarde avec diminution de la durée de la diastole, ce qui peut aider à réduire l'évolution d'un remodelage cardiaque susceptible de mener à une insuffisance cardiaque. Le dispositif comprend à cet effet une sonde pourvue d'une électrode implantée sur le nerf vague et un générateur délivrant des impulsions VNS sur cette électrode.

Lorsque le nerf vague est stimulé de façon synchrone avec le rythme cardiaque, le dispositif peut comprendre une ou plusieurs sondes cardiaques, par exemple une ou plusieurs sondes endocavitaires ou implantées dans le réseau coronarien permettant le recueil des ondes de dépolarisation cardiaque (électrogramme EGM), et éventuellement la délivrance d'impulsions de stimulation du myocarde (stimulation des cavités ventriculaire et/ou auriculaire), en supplément de la stimulation VNS appliquée séparément sur le nerf vague.

D'autres moyens de détection du rythme cardiaque peuvent être utilisés, par exemple par recueil d'un électrocardiogramme (ECG) sous-cutané au moyen d'un dispositif implantable dédié à la seule stimulation VNS, donc qui ne comprend pas de moyens de délivrance d'impulsions au myocarde et de ce fait ne nécessite pas de sonde cardiaque.

Le point de départ de l'invention résulte d'une double constatation.

En premier lieu, l'application d'une stimulation VNS délivrée, à un délai éventuel près, en synchronisme avec les ondes R de l'EGM ou de l'ECG (l'onde R correspondant au début de la dépolarisation du ventricule) a pour conséquence d'induire une modification de l'intervalle RR des cycles cardiaques successifs. Plus précisément, l'intervalle RR augmente avec l'énergie de la stimulation délivrée sur le nerf vague, car une énergie de stimulation VNS élevée a sur la fonction cardiaque un effet inhibiteur se traduisant par un ralentissement du rythme (mesuré par l'intervalle RR ; inversement, une faible énergie de stimulation VNS aura une incidence moindre sur la fonction cardiaque, avec un rythme beaucoup plus proche du rythme sinusal naturel observable en l'absence de stimulation VNS. Par ailleurs, l'évolution de la variabilité sinusale spontanée (VSS) est un indicateur très pertinent pour évaluer l'état clinique d'un patient en situation ou en risque d'insuffisance cardiaque : une diminution de la VSS révèle une aggravation de la pathologie d'insuffisance cardiaque, alors qu'inversement une augmentation de la VSS traduit une amélioration de l'état général du patient.

Le US 2007/0233194 A1 décrit un dispositif d'électrostimulation VNS avec un générateur d'impulsions synchronisé sur le rythme cardiaque et/ou respiratoire du patient. Le dispositif comprend également des moyens d'analyse de divers paramètres physiologiques et cardiaques, notamment la variabilité du rythme cardiaque (HRV). Le séquencement des impulsions VNS, en particulier l'instant d'application de la première impulsion de la salve, peut être modulé par le générateur de façon prédéterminée ou aléatoire.

Les US 2009/0088817 A1, US 2011 /0118802 A1 et US 2013/0131746 A1 décrivent d'autres exemples de stimulateurs VNS où certaines caractéristiques de la stimulation peuvent varier de façon prédéterminée ou aléatoire.

L'idée de base de l'invention est d'appliquer une stimulation VNS à un patient en situation d'insuffisance cardiaque en modulant cycle-à-cycle l'énergie de cette stimulation de manière à induire des variations de l'intervalle RR d'un cycle à l'autre, alors même que le rythme sinusal aurait été constant en l'absence de stimulation VNS. Cette variabilité sinusale neurologiquement induite (ci-après VSNI) ne sera appliquée que pendant un certain temps, jusqu'à ce que le coeur montre qu'il a retrouvé une variabilité sinusale spontanée accrue, révélant une amélioration de la pathologie d'insuffisance cardiaque ; la VSNI est alors interrompue, et elle pourra reprendre ultérieurement s'il s'avère que la VSS diminue à nouveau, jusqu'à franchir un seuil prédéfini.

Selon une caractéristique de l'invention, la VSNI est de type stochastique, c'est-à-dire non déterministe, afin d'éviter la compensation de l'excitation du nerf vague par une boucle physiologique impliquant le système nerveux central, ce qui aurait pour effet de réduire l'effet bénéfique résultant de la VSNI.

Cette modulation stochastique est obtenue en contrôlant à chaque cycle cardiaque la délivrance des impulsions des salves VNS par une fonction aléatoire ou pseudo-aléatoire de type "pile ou face" appliquée à la délivrance de chacune des impulsions de chacune des salves, ce qui évite toute compensation potentielle par un mécanisme physiologique. L'énergie VNS délivrée sur le nerf à chaque cycle cardiaque variera ainsi entre zéro (aucune impulsion délivrée) et un maximum (la totalité des impulsions initialement prévues de la salve sont délivrées).

De ce fait, à chaque cycle cardiaque est appliquée sur le nerf vague une énergie différente, non prévisible, qui provoque par voie de conséquence une modulation imprévue de l'intervalle RR dans une gamme définie au préalable entre une valeur nulle (pas d'altération de l'intervalle RR) et une valeur maximale, choisie de manière que la VSNI n'excède pas une valeur qui pourrait produire des effets délétères, notamment arythmogènes, pour le patient.

Plus précisément, l'invention propose un dispositif médical implantable actif pour le traitement de l'insuffisance cardiaque avec stimulation du nerf vague synchrone à l'activité cardiaque, ce dispositif comprenant, comme enseigné par le US 2007/0233194 A1 précité : des moyens d'analyse du rythme cardiaque aptes à recueillir un signal EGM d'électrogramme endocavitaire et à détecter une onde R de dépolarisation ventriculaire sur chaque cycle cardiaque ; et un générateur apte à produire des salves, chaque décharge comprenant N impulsions de stimulation VNS générables en succession, et le générateur étant apte à initier la production de chaque décharge éventuelle avec synchronisation sur une onde R de l'instant de délivrance de la première impulsion VNS de cette décharge. Le dispositif comprend en outre des moyens de modulation stochastique des décharges, aptes à contrôler séparément la délivrance éventuelle de chacune des N impulsions VNS de chaque décharge en fonction du résultat d'un tirage aléatoire respectif. Les moyens d'analyse du rythme cardiaque sont également aptes à évaluer un paramètre de variabilité sinusale spontanée du rythme cardiaque, et les moyens de modulation stochastique des salves sont sélectivement activables en fonction dudit paramètre de variabilité sinusale spontanée.

De façon caractéristique de l'invention, la délivrance de chaque impulsion est une délivrance éventuelle, contrôlable par inhibition sélective, ou non, de la génération de chacune desdites impulsions VNS.

De cette manière, le nombre d'impulsions VNS délivrées dans une décharge varie aléatoirement, et par voie de conséquence l'énergie de stimulation VNS contenue dans cette décharge, ce qui induit artificiellement une variabilité cycle-à-cycle de l'intervalle RR.

Par ailleurs, les moyens d'analyse du rythme cardiaque sont également aptes à évaluer un paramètre de variabilité sinusale spontanée du rythme cardiaque, et les moyens de modulation stochastique des décharges sont sélectivement activables en fonction de ce paramètre de variabilité sinusale spontanée.

En particulier, les moyens d'analyse du rythme cardiaque peuvent comparer le paramètre de variabilité sinusale spontanée à un seuil minimal prédéterminé, les moyens de modulation stochastique des décharges étant sélectivement activables lorsque ce paramètre est inférieur audit seuil minimal prédéterminé.

Le paramètre de variabilité sinusale spontanée du rythme cardiaque est de préférence une mesure de la variabilité de l'intervalle RR, en particulier une mesure du groupe formé par : l'écart-type, sur une période d'enregistrement donnée, des intervalles RR ; l'écart-type, sur une période d'enregistrement donnée, de la moyenne des intervalles RR sur des segments temporels de durée prédéterminée ; et la moyenne quadratique des variations de durée entre intervalles RR consécutifs.

De préférence, les amplitudes respectives des impulsions VNS susceptibles d'être délivrées par le générateur sont toutes égales, de même que leurs largeurs respectives, ainsi que les intervalles de temps respectifs séparant les instants d'application éventuelle de deux impulsions VNS successives susceptibles d'être délivrées par le générateur à chaque décharge, que leur délivrance par le générateur soit on non inhibée par les moyens de modulation stochastique.

Le nombre N des impulsions VNS susceptibles d'être délivrées par le générateur à chaque décharge est avantageusement compris entre 1 et 5. On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue générale de présentation du dispositif de l'invention, montrant le générateur, le myocarde et le nerf vague ainsi que les sondes utilisées.
La Figure 2 est une vue schématique par blocs correspondant aux principales fonctionnalités du générateur du dispositif de l'invention.
Les Figures 3a à 3c sont des figures explicitant la manière dont est mise en oeuvre l'invention, au moyen de différents chronogrammes montrant sur une même ligne temporelle une succession de dix cycles cardiaques, avec les ondes R détectées et les salves d'impulsions de stimulation VNS appliquées.
La Figure 4 est un organigramme résumant les différentes étapes de mise en oeuvre de la technique d'application d'une thérapie VNS selon l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur VNS connu.

Un tel stimulateur comprend un microprocesseur programmable pourvu de circuits pour mettre en forme et délivrer des impulsions de stimulation à des électrodes implantées. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

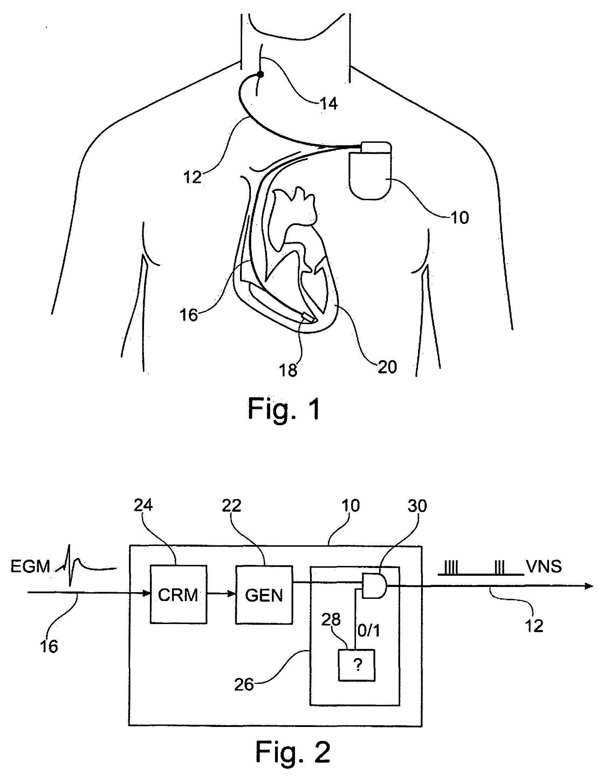

Sur la Figure 1, la référence 10 désigne le boitier d'un générateur implantable de stimulation du nerf vague. Cette stimulation est délivrée par une sonde 12 portant à sa partie distale une électrode implantée sur le nerf vague 14 et susceptible de stimuler celui-ci par application de salves d'impulsions produites par le générateur 10.

Pour permettre la délivrance d'impulsions VNS en synchronisme avec le rythme cardiaque, le générateur 10 dispose également d'une sonde cardiaque 16 pourvue à son extrémité distale d'une électrode 18 de recueil de l'activité électrique du myocarde 20. Cette sonde recueille des signaux d'électrogramme endocavitaire EGM qui permettront de piloter le générateur 10 afin que celui-ci délivre au nerf vague 14 des impulsions de stimulation VNS au même rythme que les battements cardiaques et au moment le plus approprié de l'onde de dépolarisation cardiaque.

La Figure 2 illustre de façon schématique les principales fonctionnalités du générateur 10 du dispositif de l'invention.

Celui-ci comprend un circuit générateur 22 apte à produire des salves d'impulsions VNS délivrées au nerf vague via la sonde 12. Le circuit générateur 22 est commandé par un circuit CRM 24 de gestion du rythme cardiaque, recevant en entrée le signal EGM délivré par la sonde 16.

Une première fonction du circuit CRM 24 consiste à piloter le générateur 22 de manière à délivrer des décharges d'impulsions VNS en synchronisme avec le rythme cardiaque, ce dernier étant décrit et suivi par des marqueurs correspondant aux instants de survenue de l'onde R, représentative du pic de dépolarisation spontanée des ventricules.

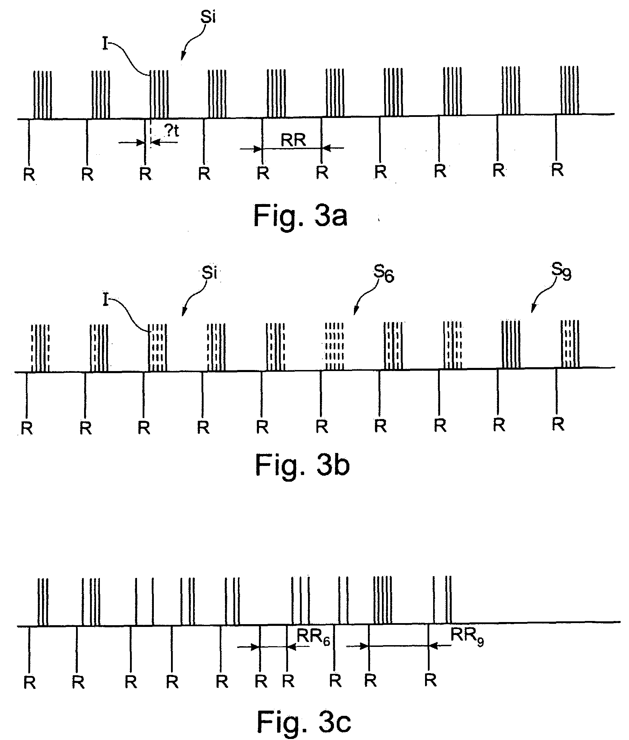

La Figure 3a illustre la position de ces marqueurs R, dont le rythme, mesuré par les durées des intervalles RR successifs, est sensiblement régulier. On a également représenté sur ce chronogramme les salves Sᵢ d'impulsions VNS, ces salves étant appliquées avec ou sans délai après l'onde R (dans l'exemple illustré, elles sont appliquées avec un délai Δt).

Chaque salve Sᵢ est constituée d'une pluralité d'impulsions individuelles I, ici au nombre de cinq. Les impulsions ont même amplitude et même largeur, de sorte que toutes les impulsions délivrent individuellement la même énergie de stimulation VNS. Par ailleurs, également dans cet exemple, l'intervalle entre deux impulsions I successives d'une même salve est un intervalle constant.

L'invention propose de moduler les salves successivement appliquées au nerf vague en décidant, à chaque salve, de délivrer un nombre variable des impulsions initialement envisagées.

Cette technique est mise en oeuvre par le bloc 26 de la Figure 2, qui représente de manière schématique et symbolique les fonctions mises en oeuvre par l'invention, de préférence implémentées concrètement sous forme d'une routine du logiciel de pilotage du dispositif.

Le circuit de modulation 26 comporte un générateur pseudo-aléatoire binaire 28 de type "pile ou face", délivrant donc en sortie, pour chaque impulsion VNS susceptible d'être appliquée, une valeur '0' ou '1' qui commandera respectivement l'inhibition ou l'autorisation de la délivrance de l'impulsion VNS, et ce pour chacune des impulsions I de chacune des salves Sᵢ (fonction schématisée par la porte ET 30).

La fonction "pile ou face" peut être obtenue par exemple avec un algorithme de tirage pseudo-aléatoire d'un nombre sur N octets, le "pile" étant représenté par la valeur '0' d'un bit prédéterminé de ce nombre, et le "face" par la valeur '1' du bit de ce même nombre.

On peut utiliser par exemple un algorithme itératif définissant une suite S telle que Sₙ₊₁ = (Sₙ*16807) modulo 4294967296, avec S₀ choisi arbitrairement, par exemple S₀ étant une valeur représentant l'horloge interne d'un système ou une combinaison de cette horloge interne et d'un autre paramètre dépendant du temps. Le résultat '0' ou '1' du tirage de rang n+1 sera alors la valeur de l'un quelconque prédéterminé des bits de Sₙ₊₁.

Le résultat de cette fonction de modulation stochastique avec inhibition/autorisation de la délivrance de chacune des impulsions de chaque salve est illustré Figure 3b, avec les salves dont la délivrance a été inhibée par rapport à la Figure 3a représentées en tiretés.

L'énergie délivrée sur chaque salve Sᵢ va ainsi varier, de façon totalement imprévisible, entre un minimum et un maximum :
- le minimum correspond à une situation où toutes les impulsions de la salve ont été inhibées, comme dans le cas de la décharge S₆;
- le maximum correspond au cas où aucune des impulsions n'a été inhibée, comme dans le cas de la salve S₉.

L'énergie délivrée sur le nerf vague varie ainsi aléatoirement, pour chaque cycle, entre zéro et un maximum correspondant (dans l'exemple illustré) à cinq fois l'énergie d'une impulsion I unitaire.

Cette modulation de l'énergie de stimulation va provoquer une modulation correspondante de l'intervalle RR, comme illustré Figure 3c, entre :
- une valeur minimale : dans l'exemple illustré, l'intervalle RR₆ correspondant à l'énergie minimale (en fait nulle) de la salve S₆ où aucune impulsion n'a été délivrée ; et
- une durée maximale, correspondant (dans l'exemple illustré) à l'intervalle RR₉ pour la salve S₉.

On observe ainsi, comme on peut le voir Figure 3c, une variabilité très importante, induite cycle-à-cycle, de l'intervalle RR.

La plage de variation artificiellement induite de cet intervalle RR est choisie, en fonction du nombre d'impulsions de chaque salve et de l'énergie individuelle de chaque impulsion, pour que cette variabilité induite soit supportable par le patient et ne crée pas de risque d'effets délétères, notamment ne soit pas arythmogène.

La stimulation VNS que l'on vient de décrire, avec une modulation cycle-à-cycle permettant de recréer une variabilité sinusale neurologiquement induite (VSNI), n'est appliquée que pendant une durée limitée, jusqu'à ce que l'on détermine que le coeur a retrouvé une variabilité sinusale spontanée (VSS) suffisante, révélant une amélioration de la pathologie d'insuffisance cardiaque : la VSNI est alors arrêtée, et elle pourra reprendre ultérieurement s'il s'avère que la VSS diminue jusqu'à franchir un seuil prédéfini.

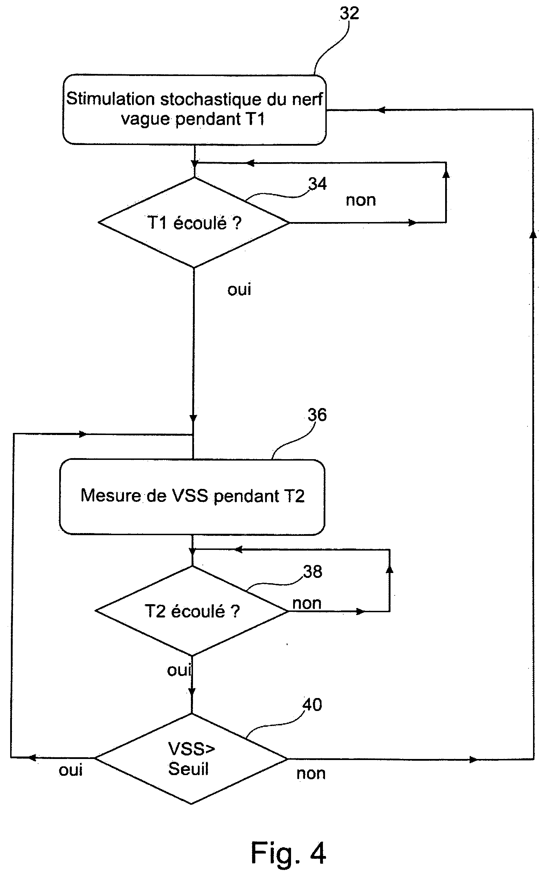

L'enchainement de ces différentes étapes est illustré sur l'organigramme de la Figure 4.

La stimulation VNS selon l'invention est appliquée (bloc 32) pendant une durée prédéterminée T1.

Une fois cette durée expirée (test 34), le dispositif évalue la VSS, pendant une durée T2 correspondant à une désactivation de la stimulation VNS et donc la reprise d'un rythme naturel (bloc 36). On peut utiliser pour la mesure de la variabilité sinusale spontanée VSS des méthodes en elles-mêmes connues telles que le calcul de :
- l'écart-type de l'intervalle RR sur toute la période d'enregistrement T2 : cette mesure est un indicateur de la variabilité globale ;
- l'écart-type de la moyenne des intervalles RR sur des segments temporels de durée prédéterminée, par exemple des segments de cinq minutes, observés sur toute la période d'enregistrement T2 : cette mesure exprime la variabilité globale des cycles de cinq minutes, c'est-à-dire une variabilité à long terme ; ou encore
- la moyenne quadratique des variations de durée entre intervalles RR consécutifs (c'est-à-dire la racine carrée de la moyenne des différences, élevées au carré, des durées entre intervalles adjacents) : cette mesure exprime en outre la variabilité de haute fréquence principalement d'origine parasympathique, modulée par la respiration.

Ces divers indices permettent d'évaluer la réponse cardiaque à la stimulation du système nerveux autonome. Après un infarctus du myocarde par exemple, la diminution de la variabilité spontanée du rythme cardiaque, exprimée par de telles variables, est un facteur prédictif de mortalité. Après expiration de la période T2 d'enregistrement avec désactivation de la stimulation VNS (test 38), la variabilité VSS mesurée pendant cette période est comparée à un seuil prédéterminé (test 40).

Si la variabilité VSS reste au-dessous de ce seuil, alors la stimulation VNS est reprise (retour au bloc 32) ; dans le cas contraire, cela signifie que l'état du patient s'est amélioré, et dès lors la stimulation VNS reste désactivée et une nouvelle période d'enregistrement T2 est initialisée (retour au bloc 36).

## Revendications

1. Un dispositif médical implantable actif pour le traitement de l'insuffisance cardiaque avec stimulation du nerf vague, VNS, synchrone à l'activité cardiaque, comprenant :
- des moyens d'analyse du rythme cardiaque (24), aptes à recueillir un signal EGM d'électrogramme endocavitaire et à détecter une onde R de dépolarisation ventriculaire sur chaque cycle cardiaque ; et
- un générateur (22), apte à produire des salves (Si), chaque salve comprenant N impulsions (I) de stimulation VNS générables en succession, le générateur étant apte à initier la production de chaque salve éventuelle avec synchronisation sur une onde R de l'instant de délivrance de la première impulsion VNS de cette salve,
- des moyens de modulation stochastique des salves (26), aptes à contrôler séparément la délivrance éventuelle de chacune des N impulsions VNS de chaque salve en fonction du résultat d'un tirage aléatoire respectif (28),
les moyens d'analyse du rythme cardiaque étant également aptes à évaluer (36) un paramètre de variabilité sinusale spontanée (VSS) du rythme cardiaque,
et les moyens de modulation stochastique des salves étant sélectivement activables (40) en fonction dudit paramètre de variabilité sinusale spontanée,
dispositif **caractérisé en ce que** la délivrance de chaque impulsion est une délivrance éventuelle, contrôlable par inhibition sélective, ou non, de la génération de chacune desdites impulsions VNS en fonction du résultat d'un tirage aléatoire respectif de manière à faire varier aléatoirement le nombre d'impulsions VNS délivrées, et par voie de conséquence l'énergie de stimulation VNS,
et ainsi induire artificiellement une variabilité cycle-à-cycle de l'intervalle RR.

2. Le dispositif de la revendication 1, dans lequel :
- les moyens d'analyse du rythme cardiaque sont également aptes à comparer (40) ledit paramètre de variabilité sinusale spontanée à un seuil minimal prédéterminé ; et
- les moyens de modulation stochastique des salves sont sélectivement activables lorsque ledit paramètre de variabilité sinusale spontanée est inférieur audit seuil minimal prédéterminé.

3. Le dispositif de la revendication 1, dans lequel ledit paramètre de variabilité sinusale spontanée (VSS) du rythme cardiaque est une mesure de la variabilité de l'intervalle RR.

4. Le dispositif de la revendication 3, dans lequel ladite mesure de la variabilité de l'intervalle RR est une mesure du groupe formé par : l'écart-type, sur une période d'enregistrement donnée, des intervalles RR ; l'écart-type, sur une période d'enregistrement donnée, de la moyenne des intervalles RR sur des segments temporels de durée prédéterminée ; et la moyenne quadratique des variations de durée entre intervalles RR consécutifs.

5. Le dispositif de la revendication 1, dans lequel les amplitudes respectives des impulsions VNS susceptibles d'être délivrées par le générateur sont toutes égales.

6. Le dispositif de la revendication 1, dans lequel les largeurs respectives des impulsions VNS susceptibles d'être délivrées par le générateur sont toutes égales.

7. Le dispositif de la revendication 1, dans lequel les intervalles de temps respectifs séparant les instants d'application éventuelle de deux impulsions VNS successives susceptibles d'être délivrées par le générateur à chaque salve (Si) sont tous égaux, que leur délivrance par le générateur soit on non inhibée par les moyens de modulation stochastique.

8. Le dispositif de la revendication 1, dans lequel le nombre N des impulsions VNS susceptibles d'être délivrées par le générateur à chaque salve est au maximum 5.

## Patentansprüche

1. Aktives implantierbares medizinisches Gerät zur Behandlung einer Herzinsuffizienz mit Stimulation des Vagusnervs, VNS, synchron zur Herzaktivität, umfassend:
- Mittel zur Analyse des Herzschlags (24), die geeignet sind, ein Signal EGM eines endokardialen Elektrogramms aufzunehmen und eine Welle R einer ventrikulären Depolarisation auf jedem Herzzyklus zu erfassen; und
- einen Generator (22), der geeignet ist, Salven (Sᵢ) zu erzeugen, wobei jede Salve N Impulse (I) zur Stimulation VNS, die aufeinanderfolgend erzeugbar sind, umfasst, wobei der Generator geeignet ist, die Produktion jeder möglichen Salve mit Synchronisierung auf einer Welle R des Zeitpunktes der Lieferung des ersten Impulses VNS dieser Salve zu initiieren,
- Mittel zur stochastischen Modulation der Salven (26), die geeignet sind, getrennt die mögliche Lieferung jedes der N Impulse VNS jeder Salve in Abhängigkeit von dem Ergebnis einer jeweiligen zufälligen Ziehung (28) zu kontrollieren,
wobei die Mittel zur Analyse des Herzschlags auch geeignet sind, einen Parameter einer spontanen Sinusvariabilität (VSS) des Herzschlags zu bewerten (36),
und wobei die Mittel zur stochastischen Modulation der Salven in Abhängigkeit von dem Parameter der spontanen Sinusvariabilität selektiv aktivierbar sind (40),
wobei das Gerät **dadurch gekennzeichnet ist, dass** die Lieferung jedes Impulses eine mögliche, durch selektive Hemmung der Erzeugung jedes der Impulse VNS in Abhängigkeit von dem Ergebnis einer jeweiligen zufälligen Ziehung kontrollierbare Lieferung oder nicht ist, um die Anzahl von gelieferten Impulsen VNS und folglich die Stimulationsenergie VNS zufällig zu variieren und somit künstlich eine Zyklus-Zyklus-Variabilität des Intervalls RR zu induzieren.

2. Gerät nach Anspruch 1, bei dem:
- die Mittel zur Analyse des Herzschlags auch geeignet sind, den Parameter der spontanen Sinusvariabilität mit einer vorbestimmten Mindestschwelle zu vergleichen (40); und
- die Mittel zur stochastischen Modulation der Salven selektiv aktivierbar sind, wenn der Parameter der spontanen Sinusvariabilität niedriger als die vorbestimmte Mindestschwelle ist.

3. Gerät nach Anspruch 1, bei dem der Parameter der spontanen Sinusvariabilität (VSS) des Herzschlags ein Maß für die Variabilität des Intervalls RR ist.

4. Gerät nach Anspruch 3, bei dem das Maß der Variabilität des Intervalls RR ein Maß der folgenden Gruppe ist: typischer Abstand der Intervalle RR über einen gegebenen Aufzeichnungszeitraum; typischer Abstand des Mittels der Intervalle RR über einen gegebenen Aufzeichnungszeitraum auf Zeitsegmenten von vorbestimmter Dauer; und quadratisches Mittel der Variationen mit einer Dauer zwischen aufeinanderfolgenden Intervallen RR.

5. Gerät nach Anspruch 1, bei dem die jeweiligen Amplituden der Impulse VNS, die vom Generator geliefert werden können, alle gleich sind.

6. Gerät nach Anspruch 1, bei dem die jeweiligen Breiten der Impulse VNS, die vom Generator geliefert werden können, alle gleich sind.

7. Gerät nach Anspruch 1, bei dem die jeweiligen Zeitintervalle zwischen den Zeitpunkten eines möglichen Anlegens von zwei aufeinanderfolgenden Impulsen VNS, die vom Generator bei jeder Salve (Si) geliefert werden können, alle gleich sind, wenn ihre Lieferung durch den Generator durch die Mittel zur stochastischen Modulation gehemmt wird oder nicht.

8. Gerät nach Anspruch 1, bei dem die Anzahl N der Impulse VNS, die vom Generator bei jeder Salve geliefert werden können, höchstens gleich 5 ist.

## Claims

1. An active implantable medical device for treatment of heart failure by stimulation of the vagus nerve, VNS, synchronous with the heart activity, comprising:
- heart rhythm analysis means (24), adapted to collect a signal of endocardial electrogram EGM and to detect a wave R of ventricular depolarization over each heart cycle; and
- a generator (22), adapted to produce bursts (Si), each burst comprising N pulses (I) of VNS stimulation able to be generated in succession, the generator being adapted to initiate the production of each possible burst with synchronization to a wave R of the instant of delivery of the first VNS pulse of each burst;
- burst stochastic modulation means (26), adapted to control separately the possible delivery of each of the N VNS pulses of each burst as a function of the result of a respective random drawing (28), the heart rhythm analysis means being also adapted to evaluate (36) a parameter of spontaneous sinusal variability (VSS) of the heart rhythm,
and the burst stochastic modulation means being selectively operable (40) as a function of said parameter of spontaneous sinusal variability,
the device being **characterized in that** the delivery of each pulse is a possible delivery, controllable by selective inhibition, or not, of the generation of each of said VNS pulses as a function of the result of a respective random drawing,
so as to randomly vary the number of VNS pulses delivered, and consequently the VNS stimulation energy,
and to hence artificially induce a cycle-to-cycle variability of the RR interval.

2. The device of claim 1, wherein:
- the heart rhythm analysis means are also adapted to compare (40) said parameter of spontaneous sinusal variability with a predetermined minimum threshold; and
- the burst stochastic modulation means are selectively operable when said parameter of spontaneous sinusal variability is lower than said predetermined minimum threshold.

3. The device of claim 1, wherein said parameter of spontaneous sinusal variability (VSS) of the heart rhythm is a measurement of the variability of the RR interval.

4. The device of claim 3, wherein said measurement of the variability of the RR interval is a measurement of the group formed by: the standard deviation, over a given recording period, of the RR intervals; the standard deviation, over a given recording period, of the average of the RR intervals over time segments of predetermined duration; and the root mean square of the variations of duration between consecutive RR intervals.

5. The device of claim 1, wherein the respective amplitudes of the VNS pulses liable to be delivered by the generator are all equal to each other.

6. The device of claim 1, wherein the respective widths of the VNS pulses liable to be delivered by the generator are all equal to each other.

7. The device of claim 1, wherein the respective time intervals separating the instants of possible application of two successive VNS pulses liable to be delivered by the generator at each burst (Si) are all equal to each other, whether the delivery thereof by the generator is inhibited or not by the stochastic modulation means.

8. The device of claim 1, wherein the number N of VNS pulses liable to be delivered by the generator at each burst is at most of 5.
